# EUROPEAN PATENT SPECIFICATION

(11) **EP 3 337 453 B1**
(45) Date of publication and mention of the grant of the patent: **22.07.2026**
(21) Application number: 16750734.2
(22) Date of filing: 03.08.2016
(51) Int. Cl.: A61Q 13/00, A61Q 19/10, A61K 8/49

(54) **LACTAM COMPOSITIONS**
LACTAM-ZUSAMMENSETZUNGEN
COMPOSITIONS À BASE DE LACTAME

(30) Priority: 20.08.2015 EP 15181846
(43) Date of publication of application: 27.06.2018
(73) Proprietor: Unilever IP Holdings B.V., 6708 WH Wageningen (NL); Unilever Global IP Limited, Wirral, Merseyside CH62 4ZD (GB)
(72) Inventor: O'KEEFFE, Joanne, Clare, Bebington Wirral Merseyside CH63 3JW (GB); PARRY, Neil, James, Bebington Wirral Merseyside CH63 3JW (GB); PRICE, Paul, Damien, Bebington Wirral Merseyside CH63 3JW (GB); STEINBERG, Peter, David, Marrickville, New South Wales 2204 (AU); THORNTHWAITE, David, William, Bebington Wirral Merseyside CH63 3JW (GB)
(74) Representative: Unilever Patent Group
(86) International application number: PCT/EP2016/068585
(87) International publication number: WO 2017/029112

(56) References cited:
- WO-A1-2007/085042
- WO-A1-2014/118240
- KRENK, O. ET AL.: "Methodology for Synthesis of Enantiopure 3,5-Disubstituted Pyrrol-2-ones", EUROPEAN JOURNAL OF ORGANIC CHEMISTRY, 17 July 2015 (2015-07-17), pages 5414 - 5423, XP002752111

## Description

The present invention relates to compositions comprising lactams. The compositions are suitable for use as anti-microbial, anti-biofilm and bacteriostatic compositions, and particularly for use as personal care compositions.

WO 2007/085042 and WO 2004/016588 disclose lactams for antimicrobial benefit and steps towards their synthesis. WO2014/118240 discloses antimicrobial compositions comprising a lactam and a hydrotope. Krenk et al. describe the synthesis of certain chiral 3,5-disubstituted pyrrol-2-ones (Eur. J. Org. Chem. 2015 pp 5414-5423).

The present invention relates to certain lactams having surprisingly advantageous toxicity profiles while retaining anti-microbial activity. These lactams have further been shown to exhibit desirable anti-bacterial and/or bacteriostatic activity. The compounds may be especially useful as the inventors believe that the lactams of the invention may be used for population control of bacterial colonies without triggering the mechanisms that are thought to lead to evolutionary resistance.

Accordingly, in a first aspect, the present invention may provide a composition comprising a lactam, wherein the lactam is a lactam selected from: 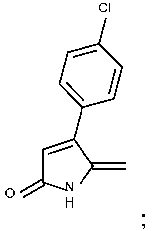 4-(4-chlorophenyl)-5-methylene-pyrrol-2-one (Ref. 488); and 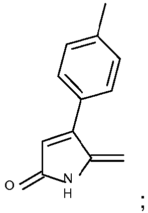 5-methylene-4-(p-tolyl)pyrrol-2-one (Ref. 491), and wherein the composition comprises a perfume.

In some cases, the lactam is 4-(4-chlorophenyl)-5-methylene-pyrrol-2-one. In some cases, the lactam is 5-methylene-4-(p-tolyl)pyrrol-2-one.

The composition may be aqueous or non-aqueous. The composition may be an emulsion.

The composition may be, without limitation, any of a personal care composition, a homecare composition, a pharmaceutical composition, or an industrial composition such as an anti-biofilm coating or paint, for example, for use in maritime environments. The composition may also be an agricultural chemical. The compositions may be suitable for use as antimicrobial, anti-biofilm and bacteriostatic compositions. Non-limiting examples of such compositions are provided herein. The compositions may also be used as additive compositions; in other words, the composition may be combined with further ingredients such as excipients to form a composition as described above.

It will be appreciated that, because of the surprisingly low toxicity, the compositions are suitably personal or homecare compositions. In some cases, the composition is a personal care composition intended for use on the skin, for example, a skin cream, a cleanser, or a serum. In some cases, the composition is a homecare composition to be used in the home, for example a laundry liquid or cleaning product.

The composition comprises a perfume ingredient, for example an encapsulated perfume. The amount of fragrance may be 0.01-1.5% wt. of the composition. Where a fragrance is encapsulated, the amount may be lower, for example, 0.01-0.5% wt.

Preferably the composition contains 0.000001 to 50% wt. lactam, more preferably 0.001 to 50% wt. even more preferably 0.01 to 5% wt., most preferably 0.01 to 2%.

### DESCRIPTION

The inventor(s) have determined that 4-(4-chlorophenyl)-5-methylene-pyrrol-2-one and 5-methylene-4-(p-tolyl)pyrrol-2-one show unexpectedly low toxicity when compared to other lactams having anti-microbial activity. This makes them especially suited for use in compositions which are used regularly by the consumer, and to compositions which may be accessible to children and domestic animals.

Without wishing to be bound to any particular theory, this may be attributed to the size and configuration of the molecules and the similarities in electronic and steric effects of the para-chloro and para-methyl substituents. These groups have properties in common when located on a phenyl ring. For example, considering their Hammett sigma constants, both have similar σᵥ (Charton's v (size) values), π Taft size parameter, and molar refractivity (polarizability) parameter values.

The inventor(s) have further determined that the compounds show desirable anti-bacterial activity. 4-(4-chlorophenyl)-5-methylene-pyrrol-2-one shows activity against both gram-positive and gram-negative bacteria, while 5-methylene-4-(p-tolyl)pyrrol-2-one shows selective activity against gram-negative bacteria.

The inventors have observed that the lactams of the composition of the invention:
- inhibit alkylquinolone (AQ) dependent quorum sensing (QS) in *P. aeruginosa.*
- inhibit PqsR in representative strains belonging to the major *P. aeruginosa* genomic groups (PAO1 and PA14 respectively).
- interact antagonistically with the LysR-type regulator PqsR (in a competitive manner without partial-agonist activity).
- do not appear to directly inhibit AQ biosynthesis but block AQ synthesis by acting as a PqsR antagonist.
- potentially interact with the co-inducer binding domain of PqsR (PqsR^{CBD}) acting an allosteric inhibitors.

Accordingly, the compositions may be suitable for long term use in population control. The inventors have found evidence that the lactams of the claimed compositions do not trigger the mechanisms that are thought to lead to evolutionary resistance

### Compositions

The compositions described herein may be compositions having anti-microbial activity. In some cases, the compositions are anti-bacterial. They may have bactericidal and / or bacteriostatic activity. The inventor(s) have observed desirable bacteriostatic activity. Accordingly, in some cases, the composition is a bacteriostatic composition.

The compositions may also prevent and / or inhibit biofilm formation. Biofilms are formed when microorganisms stick to a surface. Biofilm extracellular polymeric substances may be formed. Biofilms (also referred to as slime) present problems in industrial environments; for example, they may form in pipes in apparatus, or industrial and agricultural structures, and on boat hulls and other marine structures. Biofilms may also pose a problem in domestic environments. For example, biofilms may form in domestic appliances such as washing machines. Biofilms are also present in personal care, for example, they may form on tooth surfaces.

Compositions suitable for any and all of these applications are within the scope of the invention. In some cases, the composition is a paint or other coating. In such cases, the composition may further comprise a binder, optionally a pigment and optionally one or more conventional additives (for example, to modify surface tension, improve flow properties, improve the finished appearance, increase wet edge, improve pigment stability, etc - such additives are known in the art). The composition may comprise an aqueous solvent or an organic solvent to suit purpose.

The composition may also be used in medical applications, for example to coat equipment.

In some cases, the composition is a pharmaceutical composition. In other words, the composition may comprise a lactam selected from 4-(4-chlorophenyl)-5-methylene-pyrrol-2-one and is 5-methylene-4-(p-tolyl)pyrrol-2-one and a pharmaceutically acceptable excipient. The composition may be suitable for topical use (for example, it may be a cream or lotion), it may be suitable for ocular use (for example, it may be an used as a pharmaceutical eye drop), it may be suitable for otic use (for example, it may be used as an ear drop), it may be suitable as a mouth wash, or it may be suitable for oral administration.

In some cases, the composition is a composition suitable for use in the home (often referred to as a homecare composition). Homecare compositions include, without limitation, cleaning products, laundry detergents, and fabric conditioners. In some cases, the composition is a homecare composition, for example a laundry liquid. The composition may therefore comprise a detergent surfactant and a builder. The composition may be a fabric conditioner (also called a fabric softener) and may comprise an antistatic agent. The composition may also be a domestic cleaning product.

In some cases, the composition is a personal care composition. For example, the composition may be intended for use on the skin (for example, a cream, cleanser or serum). For example, the composition may be useful in the prevention or treatment of acne. For example, the composition may comprise one or more of dimethicone, petrolatum, a humectant such ashyaluronic acid or glycerin; and ceramide(s). In some cases, the composition is a personal care composition comprising a detergent, for example, the composition may be a face wash or shower gel.

In some cases, the composition is a contact lens cleaning fluid.

The composition may be a composition suitable for use in agriculture, for example, as a soil additive (solid or liquid).

### EXAMPLES

The following examples are provided by way of illustration and not by way of limitation.

### Preparation of 4-(4-chlorophenyl)-5-hydroxy-5-methylfuran-2(5H)-one

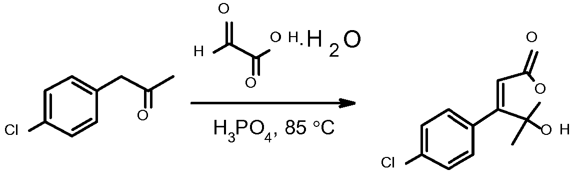

1-(4-Chlorophenyl)propan-2-one (40.00 g, 34.75 mL, 237.2 mmol), glyoxylic acid monohydrate (32.75 g, 355.8 mmol) and phosphoric acid (69.74 g, 711.7 mmol) were combined at room temperature before heating to 85 °C overnight. After cooling to room temperature, the mixture was poured into a mixture of water (500 mL) and ethyl acetate (500 mL). The layers were separated and the aqueous phase extracted with ethyl acetate (500 mL). The combined organic layers were washed with a 1:1 mixture of water and brine (2 x 500 mL), dried (MgSO₄) and concentrated under reduced pressure to yield 4-(4-chlorophenyl)-5-hydroxy-5-methylfuran-2(5H)-one (66.00 g, >100% yield) as a brown oil. The material was used in the next step without further purification.

### Preparation of 4-(4-chlorophenyl)-5-hydroxy-5-methyl-1H-pyrrol-2(5H)-one

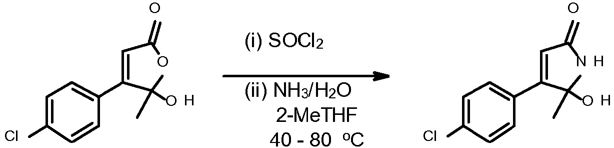

4-(4-Chlorophenyl)-5-hydroxy-5-methylfuran-2(5H)-one (66.00 g, 293.8 mmol) was dissolved in thionyl chloride (196.8 g, 120.0 mL, 1654 mmol) and heated at 40 °C for 1 hour, then 80 °C for 2 hours. The mixture was concentrated under reduced pressure and azeotroped with 2-methyltetrahydrofuran (200 mL). The residue was diluted with 2-methyltetrahydrofuran (160 mL) and this solution added to a cooled stirring mixture of 28% ammonia in water (180 mL) in 2-methyltetrahydrofuran (20 mL) at 0 °C. The mixture was warmed to room temperature and stirred overnight. Water (100 mL) and ethyl acetate (200 mL) were added and the layers separated. The aqueous phase was extracted with ethyl acetate (200 mL), and the combined organic extracts dried (MgSO₄) and concentrated under reduced pressure. Purification by dry flash column chromatography (5-60% ethyl acetate in heptane) yielded 4-(4-chlorophenyl)-5-hydroxy-5-methyl-1H-pyrrol-2(5H)-one (23.18 g, 35% yield) as a cream coloured solid.
¹H NMR (400 MHz, d₆-DMSO) 8.55 (brs, 1H), 7.88-7.83 (m, 2H), 7.51-7.46 (m, 2H), 6.37 (d, 1H), 6.32 (s, 1H), 1.45 (s, 3H)
UPLC (Basic) 1.51/5.00 min, 100% purity, M+H⁺ 224
MP 177 °C

### Preparation of 4-(4-chlorophenyl)-5-methylene-1H-pyrrol-2(5H)-one

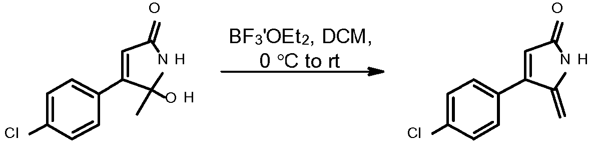

To a cooled solution of 4-(4-chlorophenyl)-5-hydroxy-5-methyl-1H-pyrrol-2(5H)-one (10.00 g, 44.51 mmol) in dry dichloromethane (100 mL) at 0 °C was added a solution of boron trifluoride diethyl etherate (8.213 g, 7.142 mL, 57.87 mmol) in dry dichloromethane (45 mL) over 15 minutes. The mixture was stirred at 0 °C, before slowly warming to room temperature and stirring for 2 hours. The reaction was quenched with ice-water (100 mL) and the layers separated. The aqueous layer was extracted with dichloromethane (100 mL), and the combined organic layers washed with a 1:1 mixture of water and saturated aqueous sodium hydrogen carbonate solution (100 mL), dried (MgSO₄) and filtered. Silica was added to the filtrate and the mixture stirred for 10 minutes before filtering through a plug of silica, washing through with dichloromethane followed by a 3:1 mixture of dichloromethane:diethyl ether. Fractions containing the desired product were combined and concentrated under reduced pressure. Upon concentration a precipitate formed, which was collected by filtration, washing with diethyl ether, to yield 4-(4-chlorophenyl)-5-methylene-1H-pyrrol-2(5H)-one (5.25 g, 57% yield) as a cream coloured solid.
¹H NMR (400 MHz, d₆-DMSO) 10.10 (s, 1H), 7.54-7.47 (m, 4H), 6.36 (s, 1H), 5.04 (t, 1H), 4.85 (s, 1H)
UPLC (Basic) 1.87/5.00 min, 100% purity, M+H⁺ 206
MP 182 °C

### Preparation of 5-hydroxy-5-methyl-4-(p-tolyl)furan-2(5H)-one

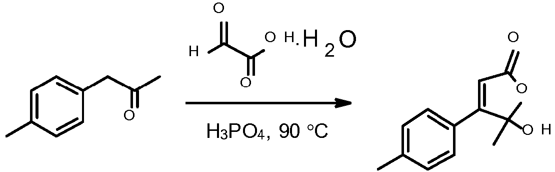

1-(p-Tolyl)propan-2-one (25.00 g, 24.00 mL, 168.7 mmol), glyoxylic acid monohydrate (23.29 g, 253.0 mmol) and phosphoric acid (49.60 g, 506.1 mmol) were combined at room temperature before heating at 90 °C overnight. After cooling to room temperature, the mixture was poured into a stirring mixture of ice-water (400 mL) and ethyl acetate (400 mL). The layers were separated and the organic phase washed with water (100 mL), dried (MgSO₄) and concentrated under reduced pressure. The mixture was azeotroped with 2-methyltetrahydrofuran (50 mL) to yield 5-hydroxy-5-methyl-4-(p-tolyl)furan-2(5H)-one (16.50 g, 48% yield) as a brown solid.

¹H NMR (400 MHz, d₆-DMSO) 7.86 (s, 1H), 7.75 (d, 2H), 7.28 (d, 2H), 6.59 (s, 1H), 2.32 (s, 3H), 1.61 (s, 3H)

### Preparation of 5-hydroxy-5-methyl-4-(p-tolyl)-1H-pyrrol-2(5H)-one

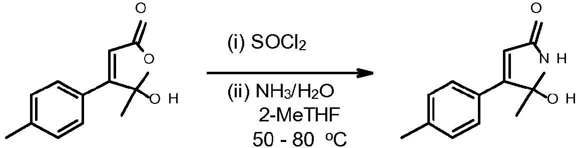

5-Hydroxy-5-methyl-4-(p-tolyl)furan-2(5H)-one (16.50 g, 80.80 mmol) was dissolved in thionyl chloride (48.06 g, 29.47 mL, 404.0 mmol) and heated at 50 °C for 1 hour, before heating at reflux for 1 hour. After cooling to room temperature, the mixture was concentrated under reduced pressure and azeotroped with 2-methyltetra-hydrofuran (2 x 50 mL). The residue was diluted with 2-methyltetrahydrofuran (60 mL) and this solution added to a cooled stirring mixture of 28% ammonia in water (55 mL, 808.0 mol) in 2-methyltetrahydrofuran (10 mL) at 0 °C. The mixture was warmed to room temperature and stirred overnight. 2-Methyltetrahydrofuran was removed under reduced pressure, and the residue diluted with water (200 mL) and diethyl ether (100 mL) and the mixture stirred for 20 minutes at room temperature. The solids were collected by filtration and stirred in water (100 mL) and diethyl ether (50 mL) at room temperature for 10 minutes. The solids were collected by filtration and washed with water, diethyl ether and dried under vacuum at 50 °C to yield 5-hydroxy-5-methyl-4-(p-tolyl)-1H-pyrrol-2(5H)-one (10.49 g, 31% yield) as a light beige solid.
¹H NMR (400 MHz, d₆-DMSO) 8.44 (brs, 1H), 7.73 (d, 2H), 7.21 (d, 2H), 6.24 (s, 2H), 2.29 (s, 3H), 1.45 (s, 3H)
¹³C NMR (400 MHz, d₆-DMSO) 170.4 (s, 1C), 161.1 (s, 1C), 139.8 (s, 1C), 129.7 (s, 2C), 128.9 (s, 1C), 128.2 (s, 2C), 119.1 (s, 1C), 87.8 (s, 1C), 26.7 (s, 1C), 21.5 (s, 1C)
UPLC (Basic) 1.41/5.00 min, 100% purity, M+H⁺ 204
MP 178 °C Decomposition

### Preparation of 5-methylene-4-(p-tolyl)-1H-pyrrol-2(5H)-one

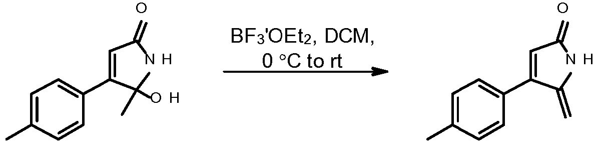

To a cooled solution of 5-hydroxy-5-methyl-4-(p-tolyl)-1H-pyrrol-2(5H)-one (8.68 g, 42.7 mmol) in dry dichloromethane (87 mL) at 0 °C was added a solution of boron trifluoride diethyl etherate (6.85 g, 5.96 mL, 55.5 mmol) in dry dichloromethane (40 mL) over 15 minutes. After 1 hour the mixture was allowed to slowly warm to room temperature. After a further 3 hours, the reaction was diluted with dichloromethane (50 mL) and ice-water (100 mL) and stirred for 10 minutes. The layers were separated and the organic layer washed with water (100 mL), a 1:1 mixture of water and saturated aqueous sodium hydrogen carbonate solution (100 mL) and brine (100 mL) and the organic layer filtered through Celite, washing with dichloromethane. Any excess water was removed by pipette before drying the filtrate (MgSO₄) and concentrating under reduced pressure to a brown solid. The solids were stirred in hot dichloromethane (120 mL) for 15 minutes before slowly cooling to room temperature and then 0 °C. The solids were collected by filtration to yield 5-methylene-4-(p-tolyl)-1H-pyrrol-2(5H)-one (3.87 g, 49% yield) as a yellow solid. Silica was added to the filtrate and the mixture stirred for 10 minutes before filtering through a plug of silica, washing through with dichloromethane and then a 4:1 mixture of dichloromethane:diethyl ether. The filtrate was concentrated under reduced pressure to yield 5-methylene-4-(p-tolyl)-1H-pyrrol-2(5H)-one (0.58 g, 7%) as a yellow solid. Total yield of 5-methylene-4-(p-tolyl)-1H-pyrrol-2(5H)-one (4.45 g, 56% yield).
¹H NMR (400 MHz, d₆-DMSO) 10.11 (brs, 1H), 7.35 (d, 2H), 7.25 (d, 2H), 6.25 (s, 1H), 5.01 (s, 1H), 4.85 (s, 1H), 2.31 (s, 3H)
UPLC (Basic) 1.83/5.00 min, 100% purity, M+H⁺ 186
MP 200 °C Decomposition

## Claims

1. An aqueous composition comprising from 0.001 to 50% wt. lactam selected from: 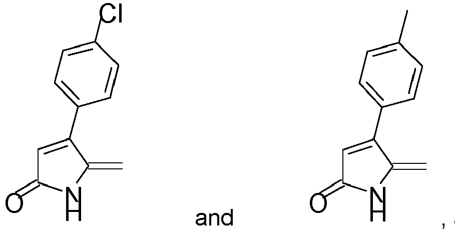 and
wherein the composition comprises a perfume.

2. The composition of claim 1, wherein the composition is an emulsion.

3. The composition of any one of claims 1 to 2, wherein the lactam is 4-(4-chlorophenyl)-5-methylene-pyrrol-2-one.

4. The composition of any one of claims 1 to 3, wherein the composition is a personal care composition, optionally wherein the composition is a skin cream, a cleanser, or a serum.

5. The composition of any one of claims 1 to 3, wherein the composition is a homecare composition, optionally wherein the composition is a laundry liquid or a cleaning product.

6. The composition of any preceding claim, wherein the composition comprises a perfume in an amount of from 0.01-1.5% wt. of the composition.

## Patentansprüche

1. Wässrige Zusammensetzung, umfassend 0,001 bis 50 Gew.-% Lactam, ausgewählt unter: 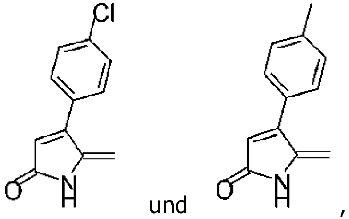 und
wobei die Zusammensetzung ein Parfüm umfasst.

2. Zusammensetzung nach Anspruch 1, wobei die Zusammensetzung eine Emulsion ist.

3. Zusammensetzung nach irgendeinem der Ansprüche 1 bis 2, wobei das Lactam 4-(4-Chlorphenyl)-5-methylen-pyrrol-2-on ist.

4. Zusammensetzung nach irgendeinem der Ansprüche 1 bis 3, wobei die Zusammensetzung eine Körperpflegezusammensetzung ist, wobei die Zusammensetzung gegebenenfalls eine Hautcreme, ein Reinigungsmittel oder ein Serum ist.

5. Zusammensetzung nach irgendeinem der Ansprüche 1 bis 3, wobei die Zusammensetzung eine Haushaltspflegezusammensetzung ist, wobei die Zusammensetzung gegebenenfalls ein Flüssigwaschmittel oder ein Reinigungsprodukt ist.

6. Zusammensetzung nach irgendeinem vorhergehenden Anspruch, wobei die Zusammensetzung in einer Menge von 0,01 bis 1,5 Gew.-% ein Parfüm umfasst.

## Revendications

1. Composition aqueuse comprenant de 0,001 à 50 % en poids de lactame choisi parmi : 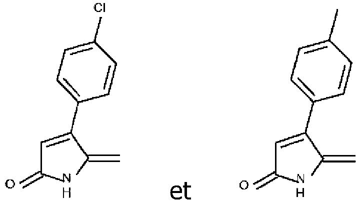 et
dans laquelle la composition comprend un parfum.

2. Composition selon la revendication 1, dans laquelle la composition est une émulsion.

3. Composition selon l'une quelconque des revendications 1 à 2, dans laquelle le lactame est la 4-(4-chlorophényl)-5-méthylène-pyrrol-2-one.

4. Composition selon l'une quelconque des revendications 1 à 3, dans laquelle la composition est une composition de soins personnels, facultativement dans laquelle la composition est une crème pour la peau, un nettoyant ou un sérum.

5. Composition selon l'une quelconque des revendications 1 à 3, dans laquelle la composition est une composition d'entretien ménager, facultativement dans laquelle la composition est une lessive liquide ou un produit de nettoyage.

6. Composition selon l'une quelconque des revendications précédentes, dans laquelle la composition comprend un parfum en une quantité de 0,01 à 1,5 % en poids de la composition.
